# EUROPEAN PATENT APPLICATION

(11) **EP 3 075 259 A1**
(43) Date of publication of application: **05.10.2016**
(21) Application number: 16162852.4
(22) Date of filing: 30.03.2016
(51) Int. Cl.: A23J 1/00, A23J 3/14, C07K 14/425, A23K 20/142, A23L 5/20, A23L 33/17

(54) **METHOD FOR REDUCING PROLAMINE CONTENT OF CEREAL PRODUCTS**

(30) Priority: 02.04.2015 US 201514677408
(71) Applicant: Prairie Gold, Inc., Bloomington, IL 61704 (US)
(72) Inventor: Shane, Philip L., Hudson, IL Illinois 61748 (US); Cheryan, Munir, Urbana, IL Illinois 61801 (US); Knapp, Shaun, Peoria, IL Illinois 61615 (US)
(74) Representative: Taruttis, Stefan Georg

(57) **Abstract**

A method for producing a low-prolamine product from a prolamine-bearing seed. Prolamine protein is extracted from the prolamine-bearing seed with an alcoholic solvent selected for solubilizing the prolamine to produce an extraction slurry. A solids fraction in the extraction slurry is removed from a prolamine fraction. The solids fraction is desolventized to provide the low-prolamine product.

## Description

### BACKGROUND OF THE INVENTION

The invention relates to the field of products from prolamine-bearing seeds, and the use of processing and selective removal of prolamine proteins to improve the nutritional quality of such products.

Prolamines are seed storage proteins with a relatively higher proportion of the amino acids proline and glutamine and a lower proportion of lysine, threonine and/or tryptophan compared to other proteins. Lysine is only 1.8 - 4% of the protein in the seed, and prolamines can constitute more than 40% of the total protein in many cereals such as corn and sorghum. This results in relatively poorer digestibility and nutritional quality of protein products derived from these seeds. Particular examples of prolamine proteins include zein in corn, gliadin in wheat, kafirin in sorghum, hordein in barley, and secalin in rye. Prolamines are typically soluble in alcohol solutions.

Corn is one of the major crops in the United States. About 25% of corn is converted to food, feed, and industrial products. Ethanol production from corn by the dry-grind process has increased exponentially. A dry-grind ethanol plant typically produces three main products: ethanol, carbon dioxide, and distillers dried grains with solubles (DDGS). Since operating costs presently are almost equal to the revenue from ethanol, profit is derived primarily from the co-products. The dry-grind ethanol process currently is a low-profit operation, and there is a need for new technologies and additional co-products to improve its profitability.

Protein forms about 10% of the dry weight of corn. It is composed of zein (a highly hydrophobic protein, soluble in isopropanol or ethanol) and glutelin (soluble in aqueous alkaline solutions), with lesser amounts of globulins and albumins. Zein is unique in that it is insoluble in water except in the presence of alcohols or high concentrations of alkali or anionic detergent.

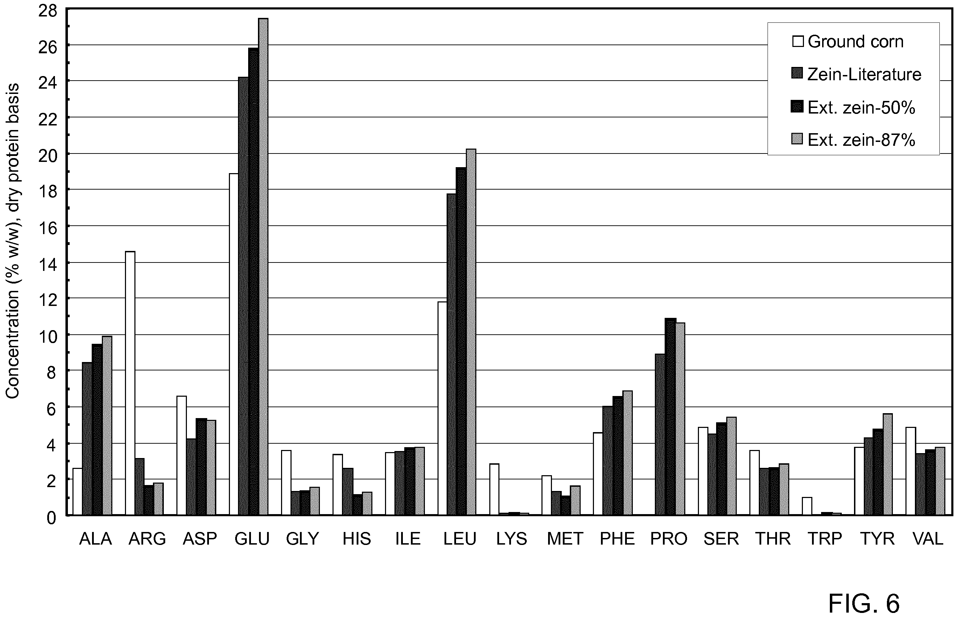

Zein has various uses in industry, but is undesirable in terms of nutritional value. FIG. 6 shows an amino acid profile comparison of ground corn obtained from a commercial corn elevator (left bar), zein as described in literature (second bar), and extracted zein having 50% purity (third bar) and 87% purity (fourth and right bar). As shown in FIG. 6, the zein is substantially devoid of lysine (LYS) and tryptophan (TRP), while the ground corn includes significant amounts of both. Lacking such amino acids, the presence of zein negatively affects the nutritional and digestible value of corn.

Various methods of producing zein from corn have been disclosed. One example method disclosed in R. Shukla and M. Cheryan, "Zein: The industrial protein from corn," Industrial Crops and Products, 13: 171-192, uses aqueous solutions of ethanol to do a first extraction of the zein from ground, flaked, or otherwise size-reduced whole corn, or corn processing byproducts from corn-gluten meal. Another example process disclosed in U.S. Patent No. 6,433,146 (incorporated herein by reference) describes a method for extracting zein from corn using aqueous ethanol, followed by a separation to remove suspended particles, and typically followed by one or more membrane separation steps using ultrafiltration and/or nanofiltration membranes to purify the zein by removal of low-molecular weight impurities.

Another method for obtaining zein from corn is disclosed in U.S. Patent No. 8,236,929, which is incorporated herein by reference. The '929 Patent discloses extracting zein from corn using aqueous ethanol, separating suspended corn solids, and purifying the resulting extract using chromatography to remove substantially purified zein. Additional corn processing methods are disclosed in U.S. Patent Nos. 7,045,607, 7,148,366, 7,667,836, and 8,236,929, which are incorporated herein by reference.

### SUMMARY OF THE INVENTION

Embodiments of the invention provide, among other things, a method for reducing the prolamine content of products from a prolamine-bearing seed to provide a low-protein product. Prolamine is extracted from raw seed or co-products of a prolamine-bearing seed with an alcoholic solvent selected for solubilizing the prolamine to produce an extraction slurry. A solids fraction in the extraction slurry is removed from a prolamine fraction. The solids fraction is desolventized to provide the low-prolamine product. Methods for using such products are also provided.

### BRIEF DESCRIPTION OF THE DRAWINGS

FIGURE 1 shows an example process for producing de-zeined meal (DZM) from an example prolamine-bearing seed, corn, according to an embodiment of the present invention;
FIG. 2 illustrates de-zeined meal (DZM) production from corn using membrane filtration according to an embodiment of the present invention;
FIG. 3 illustrates de-oiled de-zeined meal (DODZM) production from corn using membrane filtration according to an embodiment of the present invention;
FIG. 4 shows amino acid profiles of three corn products: ground corn, de-oiled meal, and de-oiled de-zeined meal in a first example experiment;
FIG. 5 shows amino acid profiles from ground corn and from de-oiled de-zeined residue in a second example experiment; and
FIG. 6 shows amino acids of ground corn as well as zein (from literature), and extracted zein having two respective protein purities.

### DETAILED DESCRIPTION

Prolamine proteins in seeds such as corn can be classified based on molecular weight (e.g., kDa), and include alpha zein, beta-zein, delta-zein, and gamma-zein. Embodiments of the present invention provide chemical and mechanical processes for reducing or removing zeins in prolamine-bearing seeds such as corn. Reducing alpha zeins and gamma zeins, for instance, improves the nutritional quality and digestibility of prolamine-bearing seeds for use in animal feed. Such reducing has been found to also improve industrial application of prolamine-bearing seeds, such as for use in wet milling or dry-grind ethanol production.

For example, particular processes disclosed herein improve an amino acid profile of products (cereal products) of prolamine-bearing seeds, such as but not limited to corn products, by selective removal of the prolamine protein (e.g., zein) which is lacking in essential amino acids lysine and tryptophan. "Low-prolamine product" or "low-prolamine meal" refers to a product of a prolamine-bearing seed having a prolamine concentration that is less than that in standard commercial feed of the prolamine-bearing seed that is used for animal feed.

In corn, for example, the removal of zein results in a significant increase in the relative proportion of lysine in the de-zeined corn product (de-zeined meal). "De-zeined corn product" or "de-zeined meal" refers to a corn product having a zein concentration that is less than that in standard commercial feed of the corn that is used for animal feed. Removed zein can include alpha-zeins, beta-zeins, delta-zein, and/or gamma-zeins. The selective removal of zein from a corn prolamine input enhances nutrient bioavailability and digestibility of the de-zeined meal while simultaneously producing a high-value zein product.

Certain embodiments of the invention provide, among other things, de-zeined corn products having an improved amino acid profile relative to a corn input, due to a selective removal of zein protein (zein). Particularly, an example de-zeined corn product has a proportionally higher content of a particular amino acid, lysine. This is due to removal of zein, which is lacking in lysine. The resulting de-zeined corn product, having reduced zein and improved lysine content, has enhanced nutrient bioavailability and digestibility. Selective removal of the zein also produces a high-value zein product. Embodiment of the invention can provide chemical extraction and mechanical processes for removing zein, including individual zein fractions, from corn, as opposed to processes such as genetic protein reduction. Alpha-zeins, for example, are desirable for film-forming applications, while beta-, delta-, and gamma-zeins are useful in applications such as encapsulation, chewing gum, etc.

Preferred embodiments will now be discussed with respect to the drawings. The drawings include schematic figures that are not to scale, which will be fully understood by skilled artisans with reference to the accompanying description. Features may be exaggerated for purposes of illustration. From the preferred embodiments, artisans will recognize additional features and broader aspects of the invention.

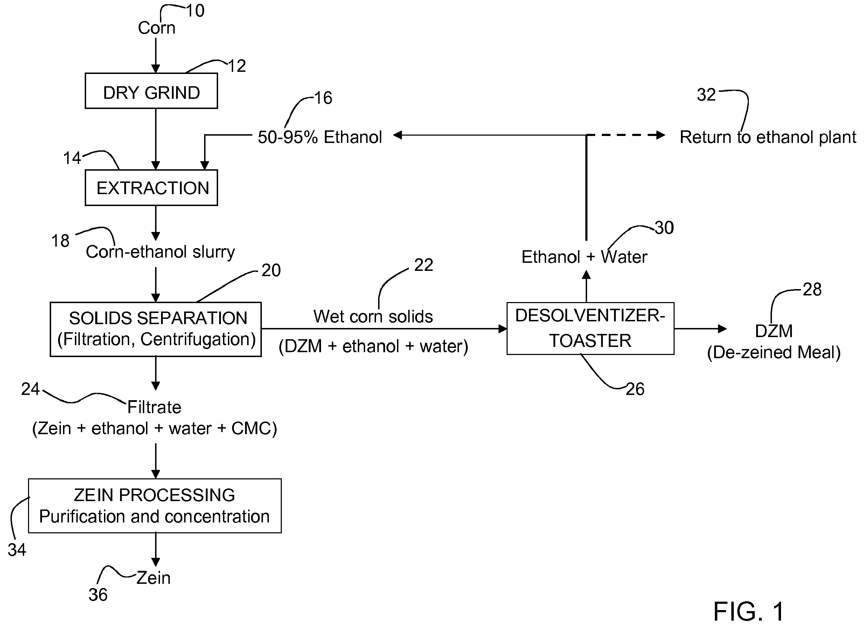

FIG. 1 shows an example process for producing de-zeined meal from a corn input by selective removal of zein proteins as a group or individual zein fractions such as alpha-zein, beta-zein, delta-zein, gamma-zein and the like. While this process can be more generally applied to prolamine-bearing seeds as inputs, a corn input is used in a particular example process. The corn input from which zein is extracted can be of multiple forms. One example is raw corn 10. Alternatively, corn co-products, such as distillers dried grains (DDGS) corn gluten meal, and corn gluten feed can be used for the corn input. For example, the raw corn 10 can, but need not in all methods, undergo one or more dry grind steps 12, e.g., dry grinding, dry milling, or flaking the corn, to provide a corn co-product such as dry ground corn, dry milled corn, flaked corn, and corn grits. If the corn input is wet milled before selective removal of zein, for example, to remove corn germ and oil and/or fiber, the corn input should be partially dried to remove water that was added during steeping or soaking or processing the corn.

Next, in an extraction step 14, zein is selectively extracted from the corn input after appropriate preparation (step 12). The extraction takes place using a delivered alcoholic solvent (i.e., a solution including alcohol that is used as a reagent) 16 that is selected or adjusted (e.g., by alcohol type, remainder, and/or concentration) for solubilizing zein. Zein fractions are defined based on solubility. For example, alpha-zein is soluble in, for example, 95% ethanol (volume/volume or v/v) or 85% isopropanol, while beta-zein is insoluble in 95% ethanol (v/v) but soluble in, for example, 60% ethanol. The relative proportions of alpha-, beta-, delta-, and/or gamma-zeins can be adjusted by selecting or adjusting the ethanol concentration in the extraction solvent. As a non-limiting example, 70% ethanol (v/v) extracts all of the individual zeins. By contrast, 88%-95% ethanol (v/v) preferentially extracts alpha-zein.

In some example embodiments, the alcohol concentration in the alcoholic solvent is selected or adjusted to target one or more particular zeins (e.g., alpha-zein, beta-zein, gamma-zein), and after zein extraction, the alcohol concentration in the alcoholic solvent is re-adjusted (e.g., raising or lowering) to target a different one or more of the zeins. The (newly targeted) zein is extracted in an additional zein extraction step (not shown) using the re-adjusted alcoholic solvent. This multistep zein extraction can include two or more extractions, and can extract targeted zeins in any desired order.

In general, the alcoholic solvent 16 can be, for instance, aqueous alcohol containing 50%-95%, and more preferably 60%-90%, by volume of alcohol. The remaining portion can be, for instance, water. An example ratio of the alcoholic solvent 16 and the (e.g., corn) input in an extraction solution is between 10:1 and about 2:1 by weight. In some example methods, the alcoholic solvent 16 is a mixture of ethyl alcohol (ethanol) and water. In other example methods, the alcoholic solvent 16 is a mixture of isopropanol and water. In still other example methods, the alcoholic solvent 16 is a mixture of ethyl alcohol, isopropanol, and water. Example extraction temperature ranges during the extraction step 14 are between 5 °C and 65 °C, preferably between 25 °C and 65 °C, and more preferably about 50 °C. An example extraction time is a period of between 0.5-3.0 hours, preferably between 0.5-2.0 hours.

If ethanol is the reagent used, as shown in the example alcoholic solvent 16 in FIG. 1, conventional dry and wet mill plants may provide a required reagent supply for implementing the method. However, ethanol or other alcohol from any source may be used. Preferably, the alcohol (e.g., ethanol or isopropanol) is the only reagent used during the extraction step 14. Other reagents could be added to improve extraction of zein, e.g., acids such as mineral acids or organic acids, alkali such as sodium lrydroxide, disulfide-disrupting chemicals such as bisulfite, thiols, and the like.

Extracting the zein from the corn input during the extraction step 14 selectively removes the zein from the corn input, e.g., by solubilizing the zein. Extracting the zein using the alcoholic solvent 16 produces an extraction slurry 18 (also referred to as an extraction solution). The extraction slurry 18 can include, for example, oils, zein, corn micro-components (CMC), other corn solids, such as but not limited to mineral salts (of sodium, calcium, etc.) that exist in both soluble and insoluble forms, water, and alcohol. Corn micro-components can include, for example, amino acids, nitrogen compounds, free fatty acids, sugars, salts, sterols, color pigments, xanthophylls, and vitamins. In the example method shown in FIG. 1, the extraction slurry 18 is a corn-ethanol slurry, though this will vary depending on the alcoholic solvent that is used.

The removed zein can be separated in a solids separation step 20 from the extraction slurry 18 using various methods to provide a wet corn solids fraction 22 including the de-zeined meal, and a separate zein fraction 24. Example solids separation steps 20 include screening, filtration or centrifugation, or any concurrent or sequential combination of these, to separate insoluble corn solids from the (solubilized) zein. If filtration is used, for instance, the zein fraction 24 can be a filtrate. If multiple solids separation steps are used, the resulting separated solids and/or liquids during each step can be recombined or separated as needed to further provide solid/liquid separation, as will be appreciated by those of ordinary skill in the art. In some methods, the wet corn solids fraction 22 includes the de-zeined meal, alcohol (ethanol in FIG. 1), and water. The zein fraction 24 can be embodied in a clarified extraction solution that includes zein, alcohol (ethanol in FIG. 1), water, and CMC.

The wet corn solids fraction 22 is desolventized in step 26 to remove substantially all ("substantially all" referring to at least 80%) of the alcohol. The desolventizing step 26 produces de-zeined meal 28, e.g., as a desolventized corn solids fraction. The de-zeined meal 28 can be partially dried during the desolventizing step. In an example method, the desolventizing step 26 is performed in a desolventizer-toaster. The desolventizer-toaster can be used to adjust a residual moisture content of the de-zeined meal to between 3%-20%, and more preferably between 7% and 15%. Additional drying can take place concurrently to and/or after desolventizing. In an example method, desolventized and partially dried de-zeined meal is dried in a separate drier (not shown) to adjust a residual moisture content of the dried de-zeined meal to between 3%-20%. Alcohol and water 30 removed during the desolventizing and drying steps 26 can be recycled as alcohol input 16. Additional alcohol can be delivered in step 32 for further processing, e.g., ethanol can be returned to an ethanol plant.

In some example methods, the wet corn solids fraction 22 is processed to remove additional liquids before the desolventizing and drying steps 26. For example, the wet corn solids fraction 22 can pass through a screw press (not shown) or other press to squeeze out additional liquids and reduce the moisture content. These additional liquids, containing zein protein, can be combined with the zein fraction 24.

The zein fraction 24 can be processed in step 34 to provide zein. For example, the zein fraction 24 can be purified and concentrated to produce a zein concentrate 36 from the clarified extraction solution, though this is not necessary in all methods (for instance, if de-zeined meal is the only desired end product). The clarified extraction solution can be purified by one or more of membrane filtration, evaporating alcohol and water from the clarified extraction solution, or precipitating zein. An example step for precipitating zein includes adding cold water to the clarified extraction solution (zein fraction 24) to lower the alcohol concentration below about 40%.

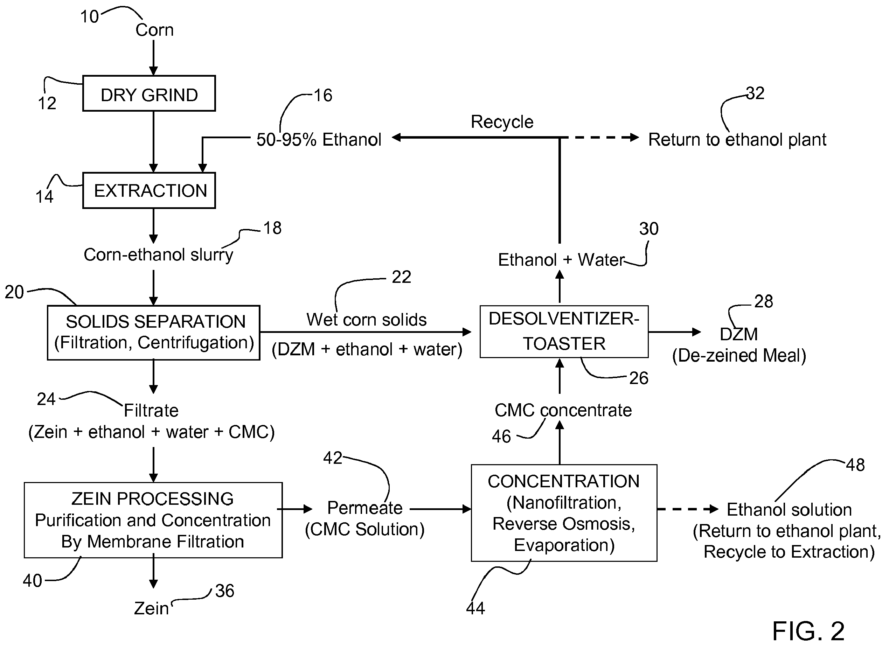

FIG. 2 shows a particular example process similar to that shown in FIG. 1 for producing de-zeined meal from corn, but particularly employing membrane filtration for zein processing and including additional downstream steps. Similar steps to those shown in FIG. 1 are referred to using like reference characters. As with the process shown in FIG. 1, the steps shown in FIG. 2 can be generally applied to other prolamine-bearing seeds to provide low-prolamine cereal products.

In the process shown in FIG. 2, the zein fraction 24, including zein, alcohol (e.g., ethanol), water, and CMC, is purified and concentrated in zein membrane filtration step 40 by membrane filtration. Example membrane filtration includes ultrafiltration and diafiltration. Diafiltration, for example, can be performed using aqueous alcohol mixtures of 60%-95%, and preferably 60%-90%, by volume. A remainder of the aqueous solution can comprise water. Example alcohols include ethanol or isopropanol. The zein membrane filtration step 40 provides a purified zein concentrate 36.

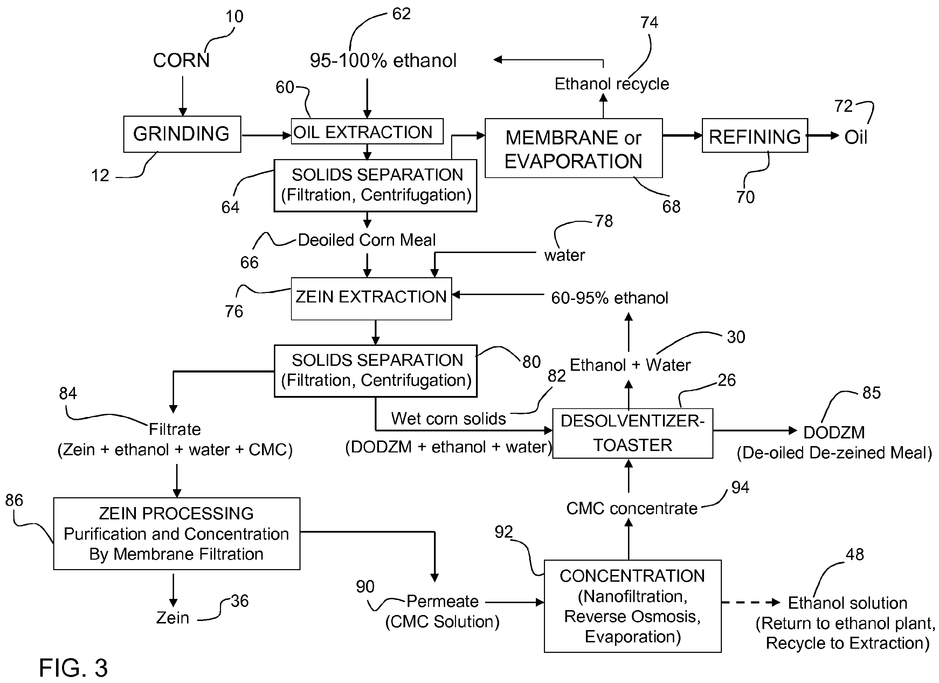

A permeate 42 from the zein membrane filtration step 40 includes the CMC in aqueous ethanol. The permeate 42 can be processed in a concentration step 44 to provide recycled alcohol (e.g., ethanol in methods where the alcoholic solvent is ethanol). The concentration step 44 separates a CMC concentrate 46 from an alcohol solution 48. Example concentration steps 44 include nanofiltration, reverse osmosis (RO), and evaporation. Recycled alcohol 48 can be used in example methods to diafilter the zein fraction 24, and/or to extract zein from the corn input in the extraction step 14. The CMC concentrate 46 can be delivered to the desolventizer/toaster 26 (or separate desolventizer and drier), and can become part of the de-zeined meal (or de-oiled de-zeined meal in the example process shown in FIG. 3).

FIG. 3 shows another example process similar to that shown in FIG. 1 and FIG. 2 for producing de-oiled and de-zeined meal from corn, employing membrane filtration for oil processing and zein processing and including additional downstream steps. As with the processes shown in FIGs. 1-2, the example process shown in FIG. 3 can be similarly applied to other prolamine-bearing seeds to provide low-prolamine cereal products. Similar steps to those shown in FIG. 1 and FIG. 2 are referred to using like reference characters.

In the process shown in FIG. 3, the corn input 10 is preferably subjected to a grinding step 12, such as dry grinding, milling, or flaking, and is first extracted in step 60 with an alcoholic solvent 62 containing 95-100% alcohol in a manner similar to that described in one or more of U.S. Patent Nos. 6,433,146, 7,148,366, 7,481,890, 7,569,671, 7,767,836, 8,344,107 and 8,344,108 which are incorporated herein by reference. The insoluble solids in the corn-solvent slurry are separated in step 64 by a suitable method such as filtration, centrifugation, screening or similar into two fractions, a corn solids phase ("de-oiled corn meal") 66 and a liquid phase. The liquid phase contains corn oil and other corn components such as protein, and other alcohol-soluble components. The liquid phase is subjected to evaporation or membrane filtration in step 68 and the recovered oil can be further refined in step 70 to produce refined oil 72, and the alcohol is recycled 74 back to the process or sent for further refining.

The de-oiled corn meal 66 is composed of about equal parts by weight of corn solids and the alcohol solvent. The example process next extracts zein in step 76, similar to step 14 in FIGs. 1-2, which requires a lower alcohol concentration. Zein extraction is done in another extractor or the same extractor by adding water 78 to the de-oiled corn meal 66 and/or a diluted alcohol solvent to adjust the alcohol concentration to 60-95% as desired for optimum zein extraction. The zein extraction step 76 can be a single step or a multistep extraction, as disclosed above. The zein extraction slurry from the zein extraction step 76 is subjected to a solids separation in step 80 (similar to step 20 in FIGs. 1-2) to separate the insoluble corn solids 82 from the liquid phase 84, e.g., a filtrate, that contains zein protein, alcohol, water and CMC. The insoluble (wet) corn solids 82 include de-oiled de-zeined meal (DODZM), ethanol, and water. These wet corn solids 82 are input to the desolventizer/toaster 26 to separate the ethanol and water 30, and output de-oiled de-zeined meal 85.

The liquid phase 84 is purified and concentrated in step 86 by membrane filtration, similar to step 40 in FIG. 2. Example membrane filtration includes ultrafiltration and diafiltration. Diafiltration, for example, can be performed using aqueous alcohol mixtures of 60%-95%, and preferably 60%-90%, by volume. A remainder of the aqueous solution can comprise water. Example alcohols include ethanol or isopropanol. The zein membrane filtration step 86 provides a purified zein concentrate 36.

A permeate 90 from the zein membrane filtration step 86 includes the CMC in aqueous ethanol. The permeate 90 can be processed in a concentration step 92, similar to step 44 in FIG. 2, to provide recycled alcohol (e.g., ethanol in methods where the alcoholic solvent is ethanol). The concentration step 92 separates a CMC concentrate 94 from an alcohol solution 48. Example concentration steps in step 92 include nanofiltration, reverse osmosis (RO), and evaporation. Recycled alcohol 48 can be used in example methods to diafilter the zein fraction 84, and/or to extract zein from the corn input in the extraction step 76. The CMC concentrate 94 can be delivered to the desolventizer/toaster 26 (or separate desolventizer and drier). The water and ethanol 30 from the desolventizer/toaster 26 can be recycled, for instance used to extract zein in the extraction step 76.

The de-zeined meal 28 (de-zeined corn product) and the de-oiled de-zeined meal 85 provide a dry feed that is lower in zein protein compared to the corn input (e.g., raw corn, or dry milled or dry ground corn). As such, the de-zeined meals 28 and 85 exhibit improved energy availability, digestibility, and/or nutritional value relative to a corn input such as raw corn. The de-zeined meals 28 and 85 also provide an improved corn input for wet milling processes. In example methods, the de-zeined meals 28, 85 are delivered as feedstock for animal feed or delivered as a corn input for a wet milling process, as described in US Patents 7,481,890, 7,569,671,767,836, 8,344,107 and 8,344,108, which are incorporated herein by reference.

Gamma-zeins, for instance, are known to be lacking in the amino acid lysine, and thus corn having higher fractions of gamma zein have a lower nutritional value. Zeins also convert to nitrogen for simple stomach animals, and removing zein from such animal feed reduces environmental consequences from excreta. For these and other reasons, the de-zeined meal 28 or de-oiled and de-zeined meal 85 can be delivered and used as an animal feed (or feed for humans) having improved nutrient digestibility and nutritional value.

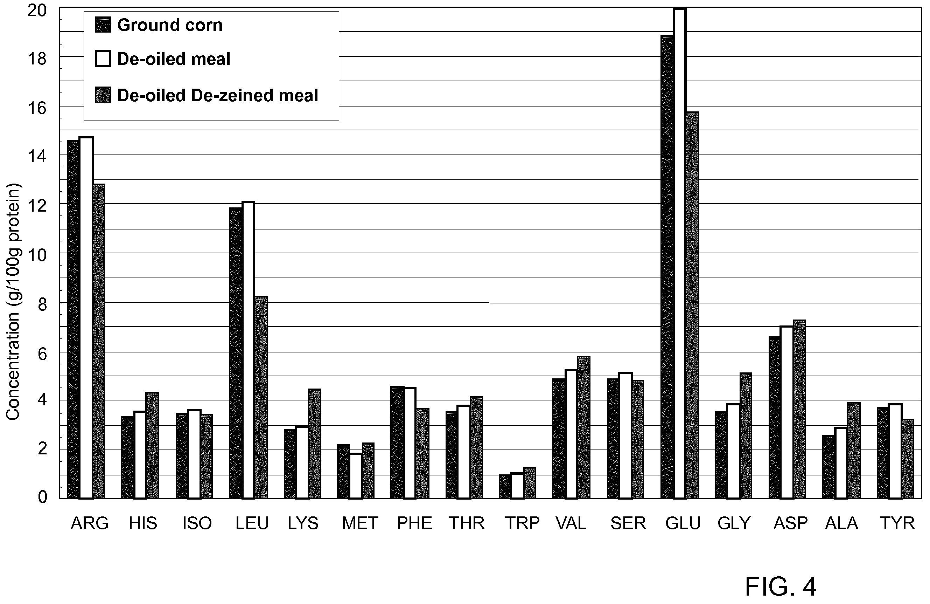

For example, FIG. 4 compares amino acid profiles of three corn products: ground corn (left bar), obtained from a commercial corn elevator, de-oiled residue (middle bar), produced using methods disclosed in U.S. Patent 7,569,671; and de-oiled and de-zeined corn meal (right bar), which was produced using the method in FIG. 3, in a first experiment. As shown in FIG. 4, the lysine (LYS) concentration (g/100g protein) is significantly greater for the de-oiled and de-zeined corn meal than for either the ground corn or the de-oiled corn residue.

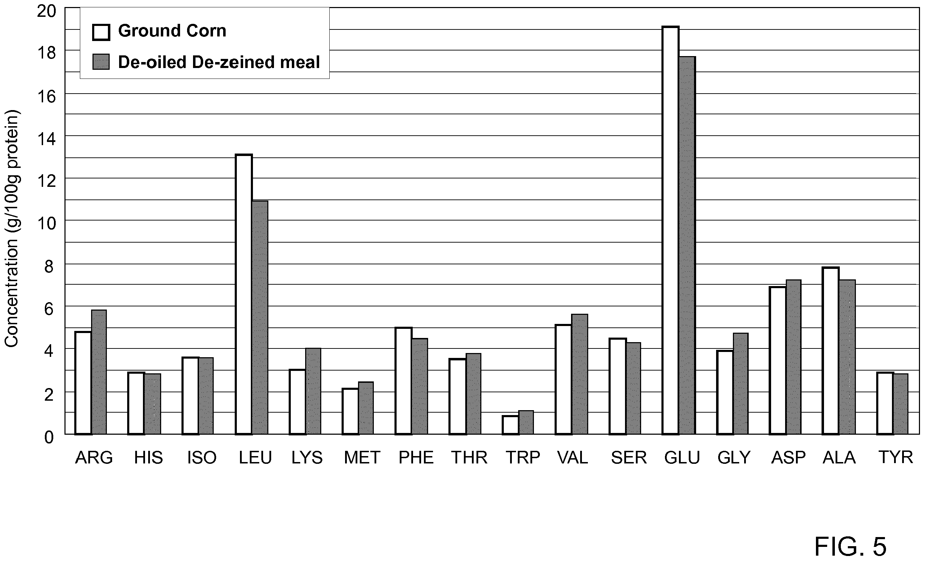

FIG. 5 compares amino acid profiles of ground corn (left bar), obtained from a commercial corn elevator; and de-oiled and de-zeined corn meal (right bar), which was produced using the method in FIG. 3 in a second experiment. Again, the lysine (LYS) concentration (g/100g protein) is significantly greater for the de-oiled and de-zeined corn meal than for the ground corn. Thus, removing zein in the de-oiled and de-zeined meal 85 provides a higher lysine content, and thus an improved amino acid profile for animal feed.

De-zeined corn product, for example, can be delivered as feedstock to monogastric livestock such as but not limited to pigs, poultry, horses, dogs, cats, rabbits, rodents, etc. as feed, and provide an improved energy availability and/or protein digestibility. De-zeined corn products can alternatively or additionally be delivered as feedstock for ruminant feed (e.g., cattle, sheep, goats, etc.), and provide improved digestibility.

Further, it is known that zeins such as alpha-zeins, beta-zeins, and gamma-zeins negatively impact wet milling processes for various reasons. For example, during wet milling processes, there is often a need for undesirable chemicals (sulfur dioxide, bisulfite) in wet mills to overcome protein disulfide bonds in the zein. Thus, de-zeined meals 28 or 85 can provide an improved corn input to a wet milling process. In addition, because of zein's adhesive nature, the presence of zein in corn processing streams causes heat exchangers and other process equipment to be fouled, resulting in down time for cleaning and reduced thermal efficiencies. Removing even partial amounts of zein can improve heat transfer and process efficiency.

De-zeined corn products can alternatively or additionally be processed to improve nutritional value, for example by milling, steam-flaking, or extrusion of the de-zeined or de-oiled de-zeined corn meal, The reduced zein content in example de-zeined corn products can reduce the energy needed for steam-flaking and grinding. Removal of zein from the de-zeined meal improves millability of the meal. As another example, the de-zeined corn meal can be processed to remove xanthophylls, for instance by using chromatographic separation. Xanthophylls can be further processed, or can be recycled.

Example methods of the present invention thus provide, among other things, a low-prolamine product and, if desired, co-products (prolamine and/or corn oil) from various forms of prolamine-bearing seeds, such as corn. The input can be prolamine-bearing seeds of multiple forms. Alcohol such as ethanol or isopropanol can be the only reagent used in an example process. In example methods where ethanol is the reagent used, conventional dry and wet mill plants could provide the required reagent supply for extraction. However, ethanol from any source could be used.

Example methods and systems can provide an add-on for existing dry mill or wet mill plants, or could be implemented in stand-alone dry mill or wet mill plants. Dry mill plants will likely gain the most benefit, as example methods can be used to extract more valuable zein from corn inputs while making use of conventional dry mill equipment and products of conventional dry mill plants.

In some particular examples, plants modified or constructed to implement example methods can partially or substantially extract zein using an ethanol extraction step with an ethanol concentration between about 60%-90%. The extraction solution containing the extracted product can be separated from other corn solids, e.g., by filtration or centrifugation. Membranes can be used to separate the zein, producing relatively pure zein. Additional membrane filtration may be used to concentrate the zein and to recover the ethanol for further use in processing, if desired. Recycled ethanol can then be used in additional zein extraction, and/or may be used in a distillation method, e.g., if ethanol product is also being produced by the plant.

It will be appreciated that although particular methods have been shown and described with respect to corn, such methods are applicable to other prolamine-bearing seeds, such as sorghum, wheat, rye and others, when used as an input. The present invention is not intended to be limited to corn processing.

While various embodiments of the present invention have been shown and described, it should be understood that other modifications, substitutions, and alternatives are apparent to one of ordinary skill in the art. Such modifications, substitutions, and alternatives can be made without departing from the spirit and scope of the invention, which should be determined from the appended claims.

Various features of the invention are set forth in the appended claims.

## Claims

1. A method for producing a low-prolamine product from a prolamine-bearing seed, the method comprising:
extracting, preferably at a temperature between 5 and 65°C and over a period of between 0.5 and 3 hours, prolamine from the prolamine-bearing seed with an alcoholic solvent selected for solubilizing the prolamine to produce an extraction slurry;
removing a solids fraction in the extraction slurry from a zein fraction; and
desolventizing the solids fraction to provide the low-prolamine product.

2. The method of claim 1, wherein the prolamine-bearing seed comprises raw corn or corn co-products;
wherein the prolamine comprises zein;
wherein the solids fraction comprises a corn solids fraction; and
wherein the low-prolamine product comprises a de-zeined corn product.

3. The process of claim 1, wherein the alcoholic solvent comprises alcohol and water, and is preferably one or more of ethyl alcohol or isopropanol mixed with water, and preferably the alcoholic solvent is aqueous ethanol or aqueous isopropanol between 60-90% by volume.

4. The method of claim 1,
wherein the prolamine-bearing seed comprises raw corn or corn co-products;
wherein the prolamine comprises zein;
wherein the solids fraction comprises a corn solids fraction; and
wherein the low-prolamine product comprises a de-zeined corn product;
wherein the raw corn or corn co-products comprise dry ground corn; and
wherein the alcoholic solvent contains 60% to 95% by volume of aqueous alcohol to remove the zein from the dry ground corn.

5. The method of claim 1, wherein said removing a solids fraction comprises one or more of filtering, centrifuging, or pressing the extraction slurry.

6. The method of claim 1, further comprising:
removing liquid from the extraction slurry after said solids separation and before said desolventizing.

7. The method of claim 1, further comprising:
before said extracting the prolamine, extracting oil from the prolamine-bearing seed with an additional alcoholic solvent having a greater concentration of alcohol that the alcoholic solvent used in said extracting the prolamine, and preferably further comprising:
separating a solids phase comprising de-oiled meal in the slurry from a liquid phase comprising the oil;
wherein said extracting the prolamine comprises extracting the prolamine from the de-oiled meal, and preferably separating the oil from the liquid phase.

8. The method of claim 1,
wherein the prolamine-bearing seed comprises raw corn or corn co-products;
wherein the prolamine comprises zein;
wherein the solids fraction comprises a corn solids fraction; and
wherein the de-zeined product comprises a de-zeined corn product;
further comprising:
delivering the de-zeined corn product as a feedstock for a wet milling plant.

9. A de-zeined corn product suitable for animal and/or human feed produced using the method of any of claims 2-8..

10. The de-zeined corn product of claim 9, wherein the de-zeined corn product has one or more of a) a greater concentration of lysine compared to the corn or corn co-product; b) a reduced pass-through nitrogen concentration when consumed by simple stomach animals; c) residual moisture content of the de-zeined corn product is between 3% and 20%; d) a lower concentration of alpha-zein compared to the corn or corn co-product; e) a lower concentration of beta-zein compared to the corn or corn co-product; and f) a lower concentration of gamma-zein compared to the corn or corn co-product.

11. The method of claim 1, wherein the prolamine comprises zein;
wherein said extracting the prolamine comprises extracting zein; and
further comprising:
before said extracting zein, selecting or adjusting a concentration of alcohol in the alcoholic solvent to extract a greater proportion of one of alpha-zein, beta-zein, or gamma-zein, and preferably further comprising after said extracting zein, re-adjusting the concentration of alcohol in the alcoholic solvent to extract a greater proportion of another of alpha-zein, beta-zein, or gamma-zein, providing a re-adjusted alcoholic solvent; and
extracting additional zein from the prolamine-bearing seed using the re-adjusted alcoholic solvent, and further preferably comprising before said extracting zein, the alcohol concentration in the alcoholic solvent is adjusted to between 60% volume/volume and 95% volume/volume to selectively remove one or more of alpha-zein, beta-zein, and gamma-zein.

12. A process for removing zein from corn, the process comprising:
extracting the zein from the corn using an alcohol solution to produce an extraction slurry including oils, zein, corn micro-components, insoluble corn solids, water, and alcohol;
separating insoluble corn solids from the extraction slurry to produce a clarified extraction solution containing zein and corn micro-components, and a separate wet corn solids fraction;
desolventizing the wet corn solids fraction to remove substantially all of the alcohol and a substantial amount of the water; and
drying the desolventized corn solids fraction to obtain de-zeined meal;
wherein a ratio between the amount of alcohol solution and the corn in said extracting is about 10:1 to 2:1 by weight;
wherein the alcohol in the alcohol solution in said extracting is about 60-95% by volume; and
wherein said extracting occurs at a temperature between 25 °C and 65 °C.

13. The process of claim 12, further comprising:
before said extracting zein, extracting oil from the corn with an additional alcoholic solvent having a greater concentration of alcohol that the alcoholic solvent used in said extracting zein, wherein said extracting oil provides a slurry, and preferably dry milling the corn before said extracting; and
separating a solids phase comprising de-oiled corn meal in the slurry from a liquid phase comprising the oil;
wherein said extracting zein comprises extracting zein from the de-oiled corn meal.

14. The process of claim 13, further comprising:
dry milling the corn before said extracting, and preferably further comprising purifying and concentrating the clarified extraction solution, such as by membrane filtering, evaporating alcohol and water, precipitating zein from the zein concentrate or diafiltering using aqueous alcohol mixtures of 60-95% by volume, to produce a zein concentrate from the clarified extraction solution.

15. The process of claim 14, wherein said purifying and separating produces a permeate; and
wherein the process further comprises processing the permeate to provide recycled ethanol.
